# EUROPÄISCHE PATENTSCHRIFT

(11) **EP 1 424 988 B1**
(45) Veröffentlichungstag und Bekanntmachung des Hinweises auf die Patenterteilung: **09.11.2005**
(21) Anmeldenummer: 02797908.7
(22) Anmeldetag: 13.07.2002
(51) Int. Cl.: A61K 7/13, D06P 1/32

(54) **AUFHELLENDES HAARFÄRBEMITTEL MIT DIREKTZIEHENDEN FARBSTOFFEN**
LIGHTENING DYE WITH DIRECT-DYEING COLORANTS
AGENT DE COLORATION CAPILLAIRE ECLAIRCISSANT COMPRENANT DES COLORANTS A ABSORPTION DIRECTE

(30) Priorität: 12.09.2001 DE 10144882
(43) Veröffentlichungstag der Anmeldung: 09.06.2004
(73) Patentinhaber: Wella Aktiengesellschaft, 64274 Darmstadt (DE)
(72) Erfinder: JAVET, Manuela, CH-1723 MARLY (CH); GÖTTEL, Otto, CH-1723 Marly (CH); DOUSSE, Christel, CH-1791 Courtaman (CH); BRAUN, Hans-Jürgen, CH-3182 Ueberstorf (CH)
(74) Vertreter: Bockhorst, Matthias
(86) Internationale Anmeldenummer: PCT/EP2002/007813
(87) Internationale Veröffentlichungsnummer: WO 2003/022232

(56) Entgegenhaltungen:
- EP-A- 0 758 547
- EP-A- 0 852 136
- WO-A-01/52802
- WO-A-97/20545
- DE-C- 19 729 080

## Beschreibung

Gegenstand der vorliegenden Erfindung sind Mittel zum Aufhellen und gleichzeitigen Färben von Haaren, welche spezielle direktziehende Farbstoffe enthalten, sowie ein Verfahren zum Färben von Keratinfasern.

Haarfärbemittel werden je nach zu färbender Ausgangshaarfarbe und gewünschtem Färbeergebnis im Wesentlichen in Oxidationsfärbemittel und nicht-oxidative Tönungen unterteilt.

Oxidative Haarfarben haben heutzutage eine wesentliche Bedeutung erlangt. Die Färbung entsteht hierbei durch Reaktion von bestimmten Entwicklersubstanzen mit bestimmten Kupplersubstanzen in Gegenwart eines geeigneten Oxidationsmittel. Oxidationsfarbstoffe, die zur Färbung menschlicher Haare eingesetzt werden, müssen zahlreiche Anforderungen erfüllen. So müssen sie physiologisch verträglich sein und Färbungen in der jeweils gewünschten Intensität liefern. Außerdem sollen die Haarfärbungen möglichst beständig gegen die Einwirkung von Licht, Dauerwellmitteln und Säuren sowie Reibung sein und unter normalen Bedingungen mindestens 4 bis 6 Wochen stabil bleiben.

Als Entwicklersubstanzen werden insbesondere 2,5-Diaminotoluol, 2,5-Diaminophenylethylalkohol, p-Aminophenol und 1,4-Diaminobenzol eingesetzt. Häufig eingesetzte Kupplerverbindungen sind: Resorcin, 1-Naphthol, 3-Aminophenol, 5-Amino-2-methylphenol, 4-Chlorresorcin und Derivate des m-Phenylendiamin. Die Verwendung dieser Substanzen kann bei bestimmten Personen zu Unverträglichkeiten führen. Einmal sensibilisierte Personen müssen diese Substanzen zur Verhinderung von Allergien meiden, so dass sie je nach gewünschtem Farbton auf direktziehende Färbemittel ausweichen müssen.

Im Vergleich zu oxidativen Färbungen besitzen nicht-oxidative Färbungen in der Regel jedoch eine geringere Haltbarkeit und einen schlechteren Farbausgleich. Zudem sind direktziehende Färbemittel in der Regel nicht in der Lage das Haar "hellerzufärben", da viele Direktzieher die zum Aufhellen benötigten Oxidationsmittel und/oder den nötigen pH-Wert von größer/gleich 9 nicht vertragen. Die Vorteile von direktziehenden gegenüber oxidativen Färbungen liegen in der im allgemeinen geringeren Haarschädigung, da normalerweise mit niedrigeren pH-Werten (kleiner 9) und ohne Oxidationsmittel gearbeitet wird. Verschiedentlich werden Direktzieher auch als Nuancierhilfen in oxidativen Färbemitteln eingesetzt. Meistens handelt es sich um Nitrofarbstoffe, die als kleine ungeladene Moleküle zwar leicht ins Haar eindringen können, aber aufgrund ihrer Grösse und fehlenden Ladung genauso leicht wieder ausgewaschen werden. Anionische Azofarbstoffe sind häufig zwar oxidationsstabil, ergeben aber im allgemeinen nur in sauren pH-Bereichen in denen man nicht gleichzeitig aufhellen kann ausreichend intensive Färbungen.

In der WO 97/20545 werden direktziehende Farbstoffe im Gelb- und Rotbereich erwähnt, die aufgrund ihrer Struktur und Ladung sowohl eine guten Haftung im Haar als auch aufgrund ihrer Stabilität den gleichzeitigen Einsatz von Oxidationsmitteln ermöglichen sollen.
Ein komplettes Färbesystem muss jedoch sowohl im Naturtonbereich als auch im Modetonbereich eine breite Palette verschiedener Farbnuancen ermöglichen, das heißt sowohl gelbe, rote und blaue Farbnuancen für den Modetonbereich als auch blonde, braune und schwarze Farbnuancen für den Naturtonbereich zur Verfügung stellen, wobei die Naturtöne gegebenenfalls auch durch Mischen verschiedener roter, gelber und blauer Farbstoffe hergestellt werden können.
Aus der WO 01/52802 sind Mittel zum Aufhellen und Färben bekannt, welche direktziehende Farbstoffe enthalten.
Aus der DE-C 197 29 080 sind nicht-oxidative Färbemittel bekannt, welche u.a. auch Kombinationen kationischer Farbstoffe enthalten können.

Aufgabe der vorliegenden Erfindung ist es daher, ein gegenüber basischen pH-Werten und Oxidationsmitteln beständiges direktziehendes Färbesystem -insbesondere für den gelben, roten und blauen Farbbereich- zur Verfügung zu stellen, welches sowohl die Nuancierung von Modetönen als auch Naturtönen erlaubt.

Es wurde nunmehr gefunden, dass diese Aufgabe durch den Einsatz von bestimmten direktziehenden kationischen Farbstoffen gelöst werden kann.

Gegenstand der vorliegenden Erfindung ist daher ein Mittel zur Aufhellung und Färbung von Keratinfasern -insbesondere Haaren-, welches dadurch gekennzeichnet ist, dass es (a) ein Oxidationsmittel sowie (b) mindestens einen blauen direktziehenden Farbstoff der allgemeinen Formel (III) und c) mindestens einen gelben direktziehenden Farbstoff der Formel (I) und/oder mindestens einen roten direktziehenden Farbstoff der Formel (II) enthält, und (d) einen basischen pH-Wert aufweist, 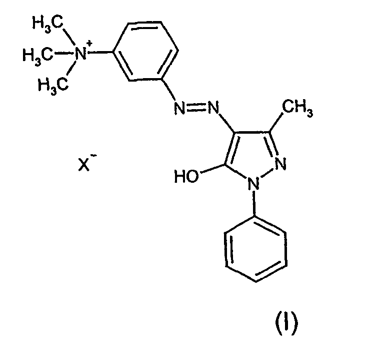 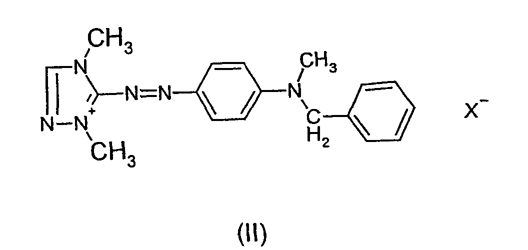 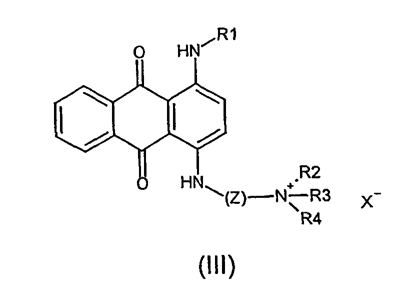 worin **R1** ein Wasserstoffatom, eine (C1-C4)-Alkylgruppe, eine Phenylgruppe oder eine (C2-C4)-Hydroxyalkylgruppe (insbesondere eine Hydroxyethylgruppe) darstellt,
**R2, R3** und **R4** gleich oder verschieden sein können und eine (C1-C4)-Alkylgruppe darstellen,
**Z** ein (C2-C6)-Alkylen-Diradikal darstellt und
**X**^{**-**} für ein Anion aus der Gruppe Chlorid, Bromid, Alkylsulfat, Arylsulfat, Sulfat oder Acetat steht.

Vorzugsweise werden Farbstoffe der Formel (III) eingesetzt, in denen
**R1** gleich einer (C1-C4)-Alkylgruppe ist, die Reste **R2** und **R3** eine Methylgruppe darstellen und **R4** gleich einer (C1-C4)-Alkylgruppe ist,
**Z** einem C2- oder C3-Alkylen-Diradikal entspricht und
**X**^{**-**} ein Anion aus der Gruppe Chlorid, Bromid oder Alkylsulfat darstellt.

Die Herstellung der Farbstoffe der Formel (III) kann in Analogie zu dem in der EP-OS 0 758 547 beschriebenen Herstellungsverfahren erfolgen. So kann zum Beispiel die Herstellung des N,N-Dimethyl-3-{[4-(methylamino)-9,10-dioxo-9,10-dihydro-1-anthracenyl]amino}-N-propyl-1-propanaminium-chlorids beziehungsweise -bromids nach dem Beispiel 3 der EP-OS 0 758 547 erfolgen, wobei jedoch anstelle des Propyljodids das Propylchlorid beziehungsweise Propylbromid verwendet wird.

Als bevorzugter gelber Farbstoff der Formel (I) ist das 3-((4,5-Dihydro-3-methyl-5-oxo-1-phenyl-1H-pyrazol-4-yl)azo)-N,N,N-trimethyl-benzenaminium-chlorid beziehungsweise das 3-[(3-Methyl-5-hydroxy-1-phenyl-1H-pyrazol-4-yl)azo]-trimethylammonio-benzol-chlorid (Basic Yellow 57) zu nennen, während als bevorzugter roter Farbstoff der Formel (II) das 3(oder5)-[[4-[Benzyl-methylamino]phenyl]-azo]-1,2-(oder 1,4)-dimethyl-1,2,4-triazolium-bromid (C.I. Basic Red 46) zu nennen ist.

Besonders bevorzugte blaue Farbstoffe der Formel (III) sind das N,N,N-Trimethyl-3-{[4-(methylamino)-9,10-dioxo-9,10-dihydro-1-anthracenyl]-amino}-1-propanaminium-methylsulfat (Handelsname: "Astrazonblau FGLN"), das N,N-Dimethyl-3-{[4-(methylamino)-9,10-dioxo-9,10-dihydro-1-anthracenyl]amino}-N-propyl-1-propanaminium-chlorid und das N,N-Dimethyl-3-{[4-(methylamino)-9,10-dioxo-9,10-dihydro-1-anthracenyl]amino}-N-propyl-1-propanaminium-bromid.

Der Gesamtgehalt an den Farbstoffen der Formeln (I) bis (III) beträgt in dem erfindungsgemäßen Färbemittel etwa 0,001 bis 8 Gewichtsprozent, vorzugsweise etwa 0,005 bis 4 Gewichtsprozent.

Zur Erweiterung der Farbpalette kann das erfindungsgemäße Färbemittel neben den Farbstoffen der Formeln (I) bis (III) zusätzlich weitere natürliche oder synthetische nicht-oxidative Farbstoffe enthalten.
Als natürliche Farbstoffe können Pflanzenfarbstoffe wie zum Beispiel Henna oder Indigo genannt werden, während als synthetische nicht-oxidative Farbstoffe Azofarbstoffe, Triphenylmethanfarbstoffe, Chinonfarbstoffe und insbesondere Nitrofarbstoffe, wie zum Beispiel 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxy-ethyl)amino]-2-nitrobenzol (HC Blue No. 10), 1-[(2,3-Dihydroxypropyl)-amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxy-propyl)amino]-2-nitrobenzol (HC Blue No. 6), 2-((4-Amino-2-nitrophenyl)-amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxy-propoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)-amino]-5-nitrobenzol (HC Yellow No. 5), 1-(2-Hydroxyethoxy)-2-[(2-hydroxyethyl)-amino]-5-nitrobenzol (HC Yellow No. 4), 1-[(2-Hydroxy-ethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis[(2-hydroxyethyl)amino]-5-nitrobenzol (HC Yellow No. 10), 4-[(2-Hydroxyethyl)-amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14) und 4-[(2-Hydroxyethyl)amino]-3-nitro-benzamid (HC Yellow No. 15), genannt werden können.

Desweiteren können neben den Farbstoffen der Formeln (I) bis (III) zusätzlich auch weitere kationische Azofarbstoffe, beispielsweise 1,4-Dimethyl-5-[(4-(dimethylamino)phenyl)azo]-1,2,4-triazolium-chlorid (C.I. 11055; Basic Red 22), 1-Methyl-4-{[methyl(phenyl)hydrazono]methyl}pyridinium-chlorid (Basic Yellow 87), 1-Methyl-4-{(E)-[methyl(4-methoxyphenyl)hydrazono]methyl}pyridinium-chlorid, 1-Methyl-4-({methyl[4-methoxy-phenyl]hydrazono}methyl)pyridinium-methylsulfat (Basic Yellow 91), 2-{[4-(Dimethylamino)phenyl]azo}-1,3-dimethyl-1H-imidazol-3-ium-chlorid (Basic Red 51), 5-{[4-(Dimethylamino)phenyl]azo}-1,2-dimethyl-1H-pyrazol-2-ium-chlorid , 1,3-Dimethyl-2-{[4-(methylamino)phenyl]azo}-1H-imidazol-3-ium-chlorid (Basic Red 109), 2-[(4-Aminophenyl)azo]-1,3-dimethyl-1H-imidazol-3-ium-chlorid, 4-{[4-(Dimethylamino)phenyl]azo}-1-methylpyridinium-chlorid und N,N-Dimethyl-4-[(E)-(1-oxido-4-pyridinyl)-diazenyl]anilin, enthalten sein.

Je nach verwendeter Farbträgermasse können in speziellen Fällen auch mit den verwendeten kationischen Farbstoffen verträgliche anionische ("saure") Farbstoffe zugesetzt werden.

Der Gesamtgehalt an natürlichen und/oder synthetischen nicht-oxidativen Farbstoffen in dem erfindungsgemäßen Färbemittel beträgt etwa 0,01 bis 15 Gewichtsprozent, insbesondere etwa 0,1 bis 12 Gewichtsprozent.

Selbstverständlich können dem erfindungsgemässen Färbemittel auch oxidative Farbstoffvorstufen, wie zum Beispiel Paraphenylendiamine, Metaphenylendiamine, Aminophenole oder 4,5-Diaminopyrazole zugesetzt werden.

Die zusätzlichen Entwicklersubstanzen und Kupplersubstanzen können in dem Färbemittel jeweils in einer Gesamtmenge von etwa 0,01 bis 20 Gewichtsprozent, vorzugsweise etwa 0,1 bis 10 Gewichtsprozent und insbesondere 0,1 bis 5 Gewichtsprozent, enthalten sein.

Zur Erhöhung der Farbintensität können erforderlichenfalls die in kosmetischen Systemen üblichen Carrier zugesetzt werden. Geeignete Verbindungen werden zum Beispiel in der DE-OS 196 18 595 beschrieben, auf die hiermit ausdrücklich Bezug genommen wird. Besonders geeignete Carrier sind zum Beispiel Benzylalkohol, Vanillin und Isovanillin.

Die vorstehend beschriebenen Farbstoffe werden zur Färbung in einer geeigneten Farbträgermasse appliziert.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffe mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,1 bis 30 Gewichtsprozent, die Verdicker in einer Menge von etwa 0,1 bis 30 Gewichtsprozent und die Pflegestoffe in einer Konzentration von etwa 0,1 bis 5,0 Gewichtsprozent.

Darüber hinaus können in dem Färbemittel noch weitere übliche Zusatzstoffe, beispielsweise Antioxidantien wie Ascorbinsäure, Thioglykolsäure oder Natriumsulfit, sowie Parfümöle, Penetrationsmittel, Puffersysteme, Komplexbildner, Konservierungsstoffe, Netzmittel, Emulgatoren, Verdicker und Pflegestoffe enthalten; sein.

Das gebrauchsfertige erfindungsgemäße Färbemittel wird unmittelbar vor Gebrauch durch Mischen der die Farbstoffe enthaltenden Farbträgermasse mit einem Oxidationsmittel hergestellt.

Als Oxidationsmittel kommen hauptsächlich Wasserstoffperoxid oder dessen Additionsverbindungen an Harnstoff, Melamin, Natriumborat oder Natriumcarbonat in Form einer 1- bis 12prozentigen, vorzugsweise einer 3- bis 6prozentigen, wässrigen Lösung, in Betracht. Das Gewichtsverhältnis zwischen Farbträgermasse und Oxidationsmittel beträgt hierbei vorzugsweise etwa 5:1 bis 1:3, insbesondere 1:1 bis 1:2. Größere Mengen an Oxidationsmittel werden vor allem bei höheren Konzentrationen an oxidativen Farbstoffvorstufen im Färbemittel, oder wenn gleichzeitig eine stärkere Bleichung der Keratinfaser (insbesondere der Haare) beabsichtigt ist, verwendet.

Des pH-Wert des gebrauchsfertigen erfindungsgemäßen Färbemittel stellt sich bei der Mischung der Farbträgermasse mit dem Oxidationsmittel auf einen pH-Wert ein, der durch die pH-Werte der Farbträgermasse des Oxidationsmittel sowie durch das Mischungsverhältnis bestimmt wird. Das gebrauchsfertige Mittel weist einen basischen pH-Wert von größer 7, vorzugsweise einen pH-Wert von 8 bis 11, auf. Die basische Einstellung erfolgt hierbei vorzugsweise mit Ammoniak, wobei jedoch auch organische Amine, zum Beispiel 2-Amino-2-methyl-1-propanol, Tris(hydroxymethyl)amino-methan, Monoethanolamin und Triethanolamin, oder Mischungen von organischen Aminen und Ammoniak sowie anorganische Basen wie Natriumhydroxid und Kaliumhydroxid Verwendung finden können. Bei zu hohen pH-Werten kann mit anorganischen oder organischen Säuren, zum Beispiel Phosphorsäure, Essigsäure, Milchsäure, Ascorbinsäure, Zitronensäure oder Weinsäure, korrigiert werden.

Anschließend trägt man eine für die Färbebehandlung ausreichende Menge, im allgemeinen etwa 60 bis 200 Gramm, dieses Gemisches auf die Keratinfaser auf und läßt das Gemisch bei etwa 15 bis 50 °C, vorzugsweise 30 bis 40 °C, etwa 10 bis 45 Minuten lang, vorzugsweise 30 Minuten lang, auf die Keratinfaser einwirken, spült sodann die Keratinfaser mit Wasser aus und trocknet sie. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird die Keratinfaser getrocknet.

Außerdem ist es möglich, bei Färbungen von unterschiedlich stark geschädigtem Haar (beispielsweise Nachfärbungen schon oxidativ gefärbter Haarpartien), auf die vorgeschädigten Haarpartien (zum Beispiel die Haarspitzen) die Farbträgermasse ohne Oxidationsmittel -pur oder nur mit einer weiteren sauren, neutralen oder basischen wässrigen Komponente verdünnt- aufzutragen, während man auf die gering oder gar nicht vorgeschädigten Haarpartien (beispielsweise den Haaransatz und die Haarlängen) die mit dem Oxidationsmittel vermischte Farbträgermasse aufträgt. Die zur Verdünnung eingesetzte wässrige Komponente kann die oben üblichen Zusätze für Lösungen, Cremes, Emulsionen oder Gelen enthalten. Dieses Verfahren ermöglicht auf die Haarbeschaffenheit abgestimmte Färbungen, die sich durch einen haarschonenden Ausgleich zwischen Ansatz und Spitzen auszeichnen, was bei der Verwendung von üblichen oxidativen Haarfärbemitteln nicht möglich ist, da zum Kuppeln der Farbstoffvorstufen immer ein Oxidationsmittel benötigt wird.

Das erfindungsgemäße Färbemittel ermöglicht Färbungen, die das komplette Farbspektrum abdecken und sich insbesondere durch ihre besondere Farbintensität und Leuchtkraft, einen guten Farbausgleich zwischen geschädigtem und ungeschädigtem Haar (zum Beispiel zwischen Haarspitzen und Haamachwuchs) sowie der sehr guten Haarschonung auszeichnen.

Die nachfolgenden Beispiele sollen den Gegenstand näher erläutern.

### Beispiele

### Beispiel 1 bis 8: Haarfärbemittel

| Farbstoff der Formel (I), (II) und/oder (III) | gemäß Tabelle 1 |
|---|---|
| Steareth-20® | 2,8 g |
| Cetylstearylalkohol | 12,0 g |
| Ethanol | 20,0 g |
| Wasser, vollentsalzt | ad 100,00 g |

Der pH-Wert wird mit 25 %igem Ammoniak auf 10 eingestellt.

5 g der vorstehenden Farbträgermasse werden mit 7,5 g einer 9 %igen Wasserstoffperoxidlösung vermischt. Das erhaltene gebrauchsfertige Haarfärbemittel wird auf Haarsträhnchen aufgetragen und mit einem Pinsel gleichmäßig verteilt. Nach einer Einwirkungszeit von 30 Minuten bei 40 °C wird das Haar mit lauwarmem Wasser gespült mit Shampoo gewaschen, mit lauwarmem Wasser gespült und sodann getrocknet.
Die Färberesultate sind in der nachfolgenden Tabelle 1 zusammengefasst.

Die in den vorliegenden Beispielen angegebenen L*a*b*-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Alle Prozentangaben in der vorliegenden Anmeldung stellen, sofern nicht anders angegeben, Gewichtsprozente dar.

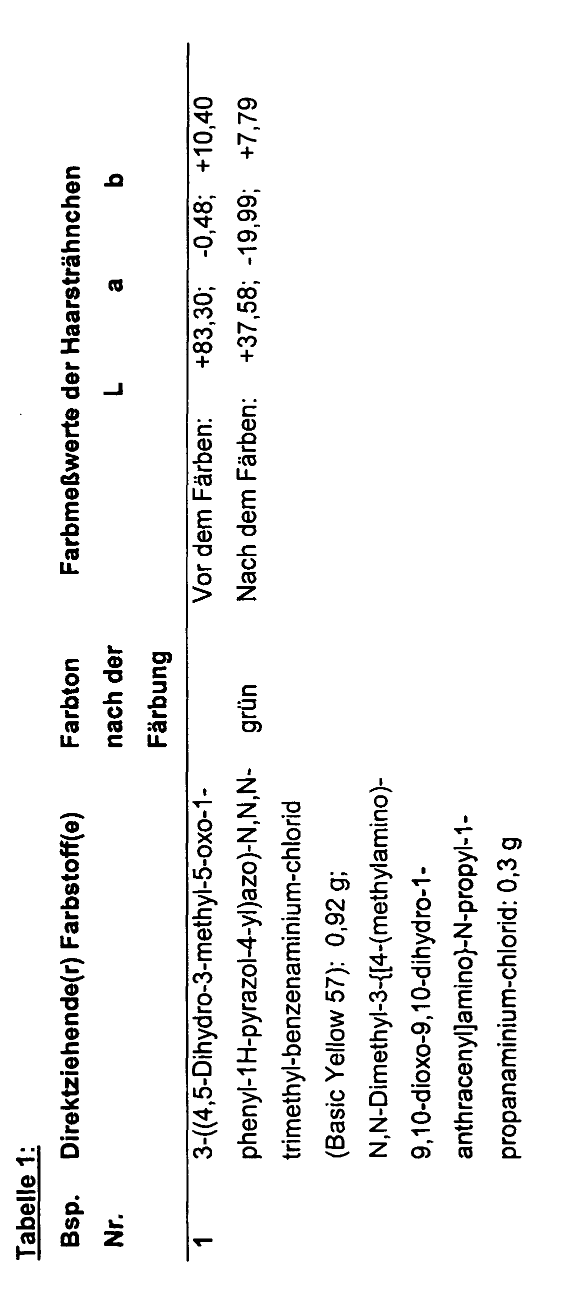

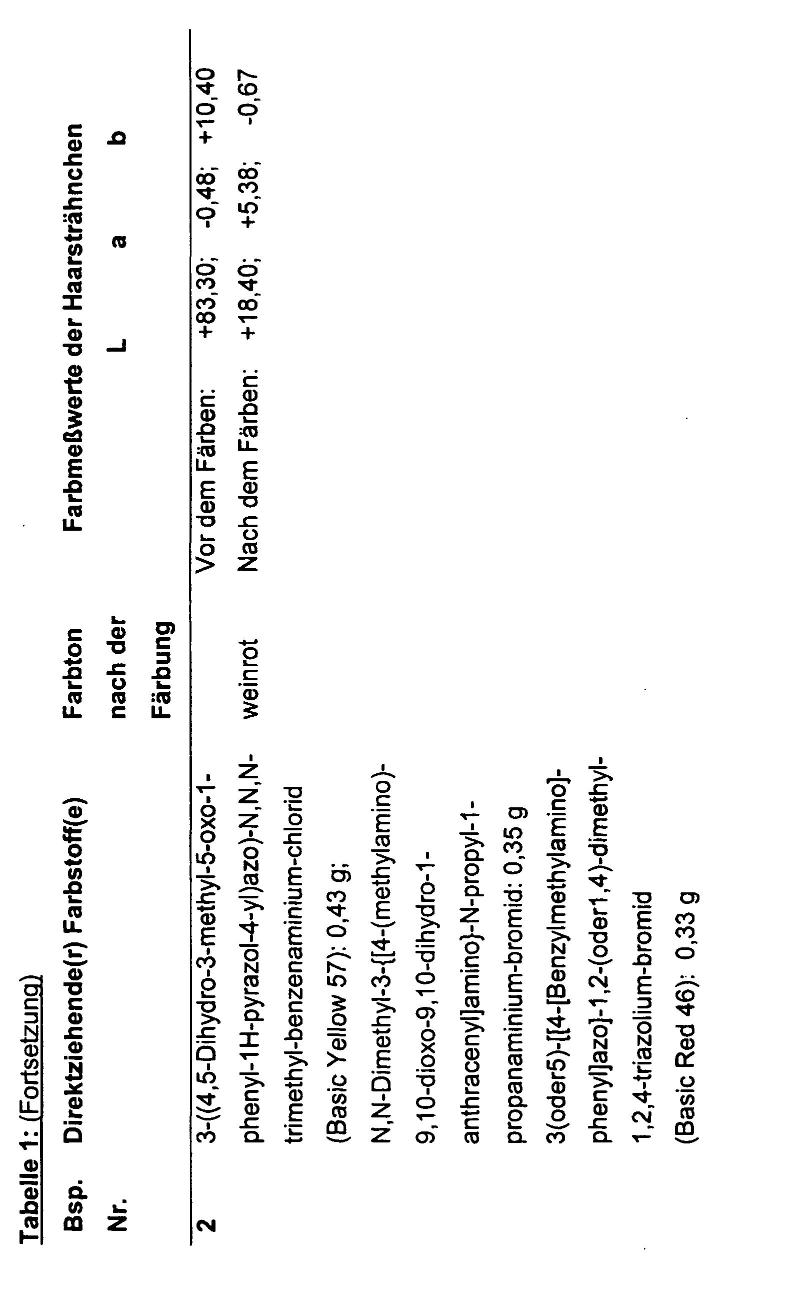

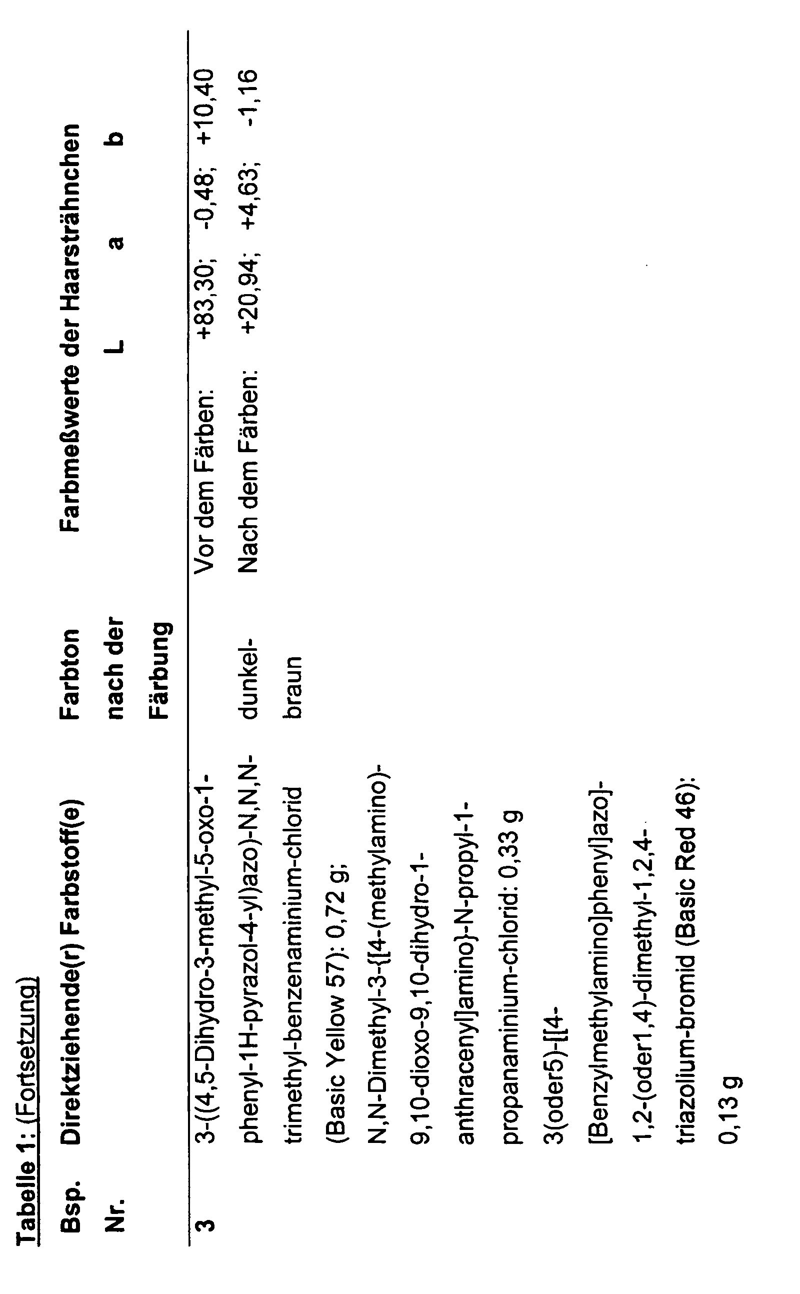

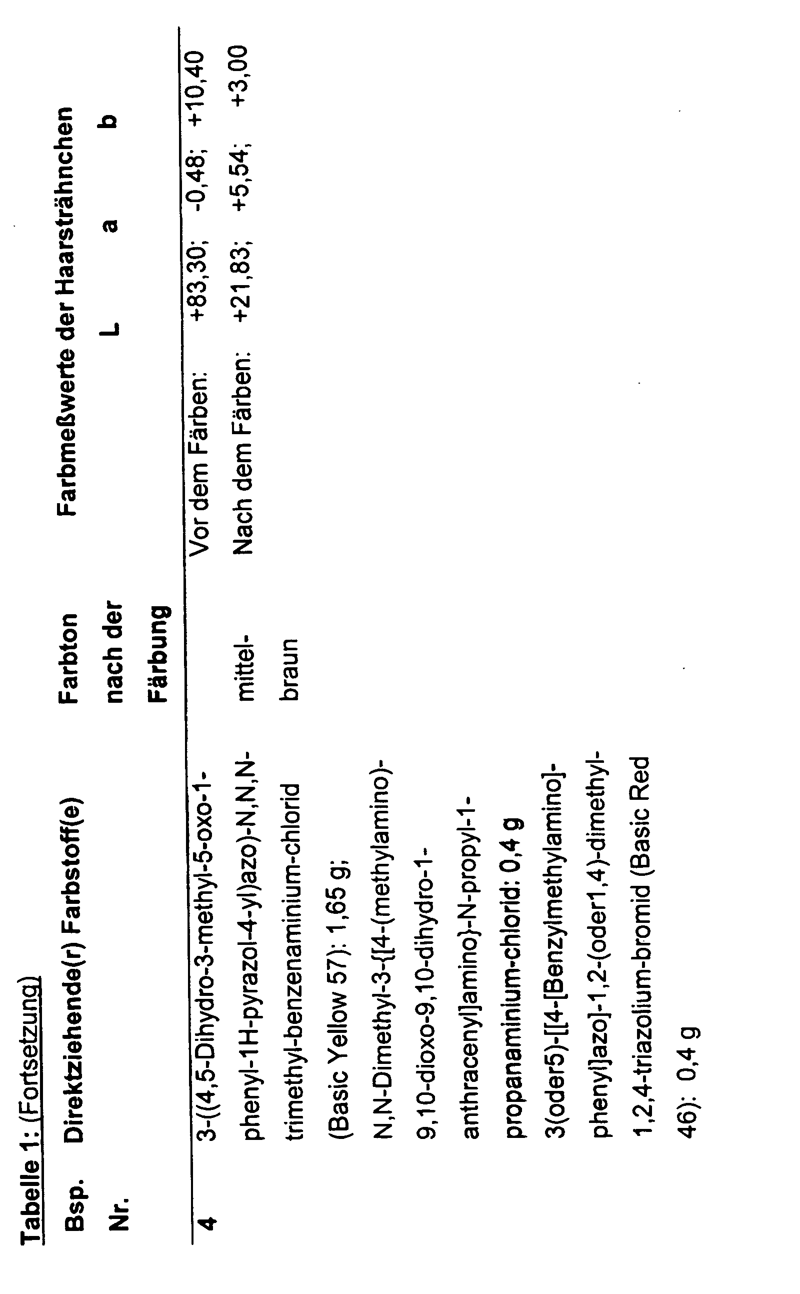

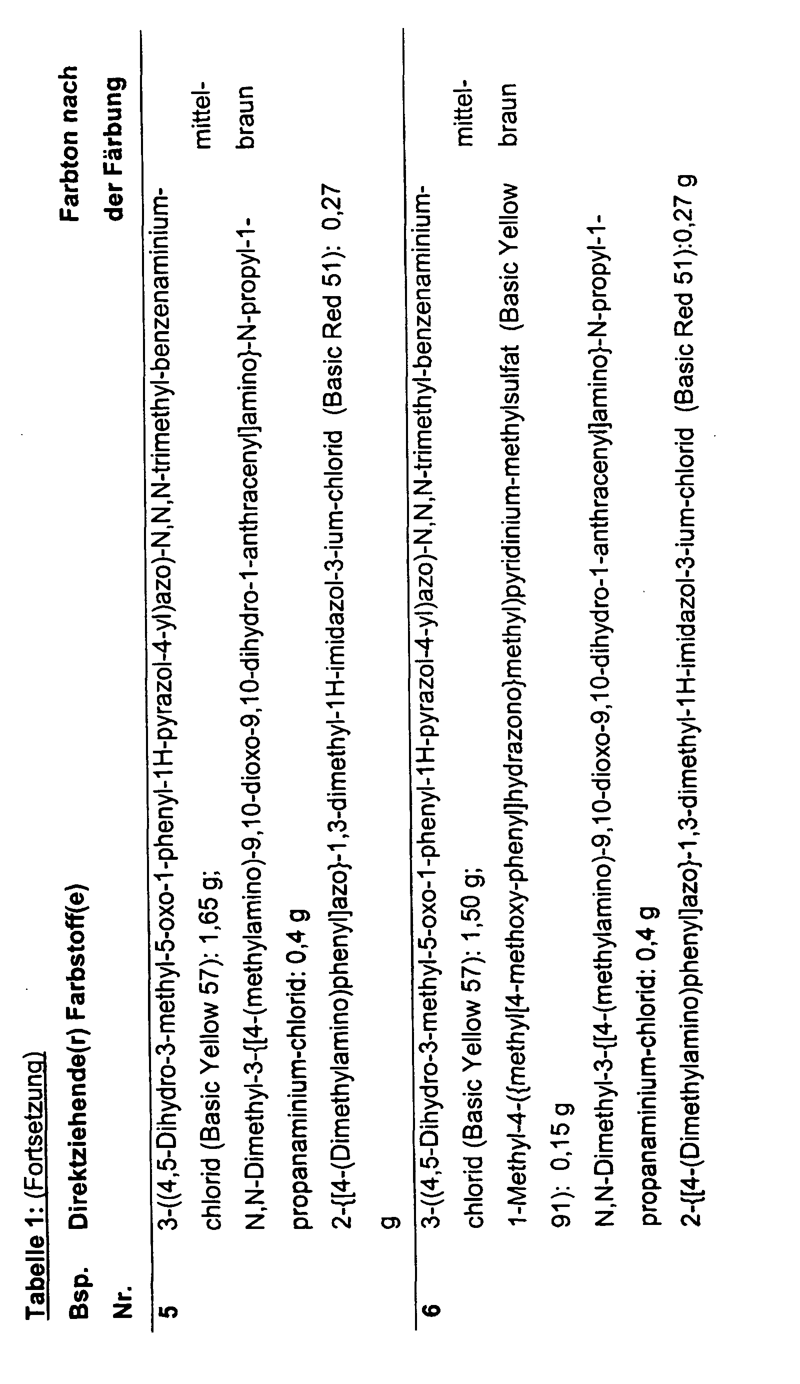

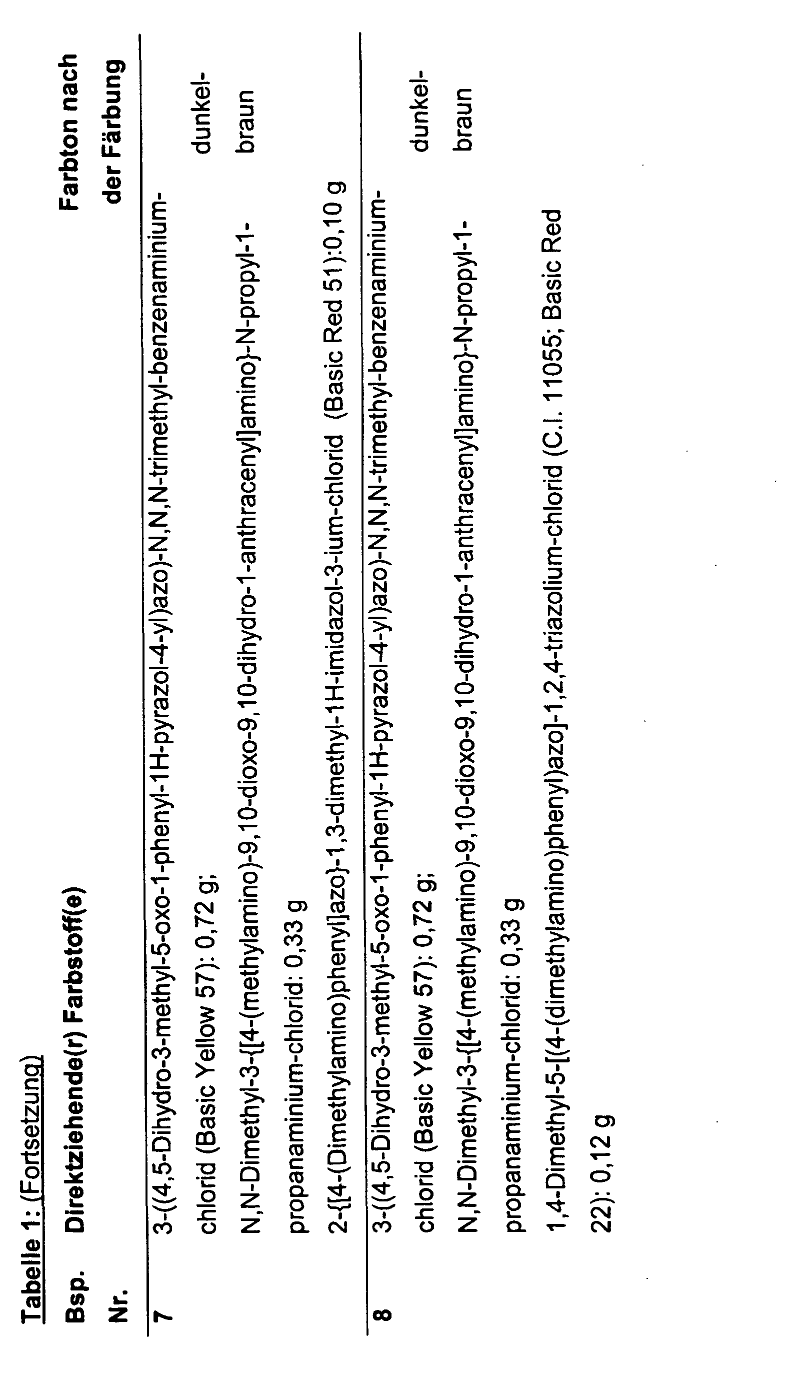

## Patentansprüche

1. Gebrauchsfertiges Mittel zur Aufhellung und Färbung von Keratinfasern, **dadurch gekennzeichnet, dass** es (a) ein Oxidationsmittel sowie (b) mindestens einen blauen direktziehenden Farbstoff der allgemeinen Formel (III) und c) mindestens einen gelben direktziehenden Farbstoff der Formel (I) und/oder mindestens einen roten direktziehenden Farbstoff der Formel (II) enthält, und (d) einen basischen pH-Wert aufweist, 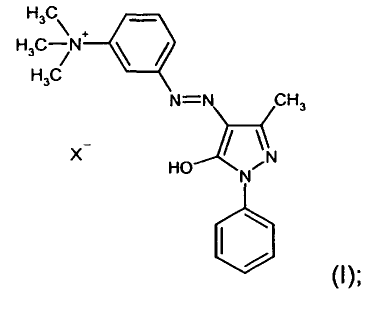 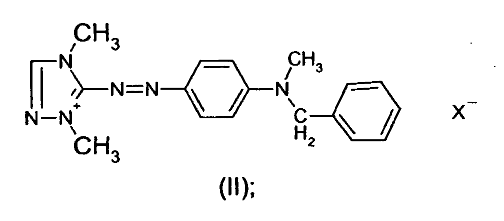 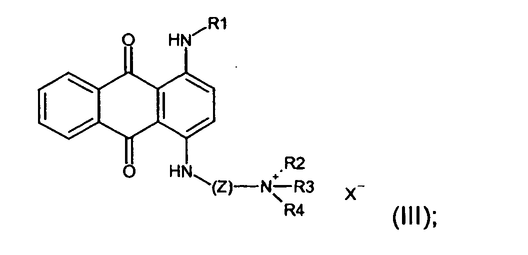 worin **R1** ein Wasserstoffatom, eine (C1-C4)-Alkylgruppe, eine Phenylgruppe oder eine (C2-C4)-Hydroxyalkylgruppe darstellt,
**R2, R3** und **R4** gleich oder verschieden sein können und eine (C1-C4)-Alkylgruppe darstellen; **Z** ein (C2-C6)-Alkylen-Diradikal darstellt und
**X**^{**-**} für ein Anion aus der Gruppe Chlorid, Bromid, Alkylsulfat, Arylsulfat, Sulfat oder Acetat steht.

2. Mittel nach Anspruch 1, **dadurch gekennzeichnet, dass** der Farbstoff der Formel (III) ausgewählt ist aus Verbindungen der Formel (III), in denen **R1** gleich einer (C1-C4)-Alkylgruppe ist, die Reste **R2** und **R3** eine Methylgruppe darstellen und **R4** gleich einer (C1-C4)-Alkylgruppe ist;
**Z** einem C2- oder C3-Alkylen-Diradikal entspricht und
**X**^{**-**} ein Anion aus der Gruppe Chlorid, Bromid oder Alkylsulfat darstellt.

3. Mittel nach Anspruch 1, **dadurch gekennzeichnet, dass** der Farbstoff der Formel (I) das 3-((4,5-Dihydro-3-methyl-5-oxo-1-phenyl-1H-pyrazol-4-yl)azo)-N,N,N-trimethyl-benzenaminium-chlorid beziehungsweise das 3-[(3-Methyl-5-hydroxy-1-phenyl-1H-pyrazol-4-yl)azo]-trimethylammonio-benzol-chlorid ist.

4. Mittel nach Anspruch 1, **dadurch gekennzeichnet, dass** der Farbstoff der Formel (II) das 3(oder5)-[[4-[Benzylmethylamino]phenyl]-azo]-1,2-(oder 1,4)-dimethyl-1,2,4-triazolium-bromid ist.

5. Mittel nach einem der Ansprüche 1 bis 4, **dadurch gekennzeichnet, dass** der Farbstoff der Formel (III) ausgewählt ist aus N,N,N-Trimethyl-3-{[4-(methylamino)-9,10-dioxo-9,10-dihydro-1-anthracenyl]amino}-1-propanaminium-methylsulfat, N,N-Dimethyl-3-{[4-(methylamino)-9,10-dioxo-9,10-dihydro-1-anthracenyl]amino}-N-propyl-1-propanaminium-chlorid und N,N-Dimethyl-3-{[4-(methylamino)-9,10-dioxo-9,10-dihydro-1-anthracenyl]amino}-N-propyl-1-propanaminium-bromid.

6. Mittel nach einem der Ansprüche 1 bis 5, **dadurch gekennzeichnet, dass** der Gesamtgehalt an den Farbstoffen der Formel (I) bis (III) gleich 0,001 bis 8 Gewichtsprozent ist.

7. Mittel nach einem der Ansprüche 1 bis 6, **dadurch gekennzeichnet, dass** es zusätzlich zu den Farbstoffen der Formeln (I) bis (III) weitere direktziehende und/oder oxidative Farbstoffvorstufen enthält.

8. Mittel nach einem der Ansprüche 1 bis 7, **dadurch gekennzeichnet, dass** das gebrauchsfertige Färbemittel einen pH-Wert von 8 bis 11 aufweist.

9. 2-Komponenten-Mittel zur Färbung von Keratinfasern, bestehend aus einer Farbträgermasse (A) und einer ein Oxidationsmittel enthaltenden Komponente (B), **dadurch gekennzeichnet, dass** die Farbträgermasse (A) (i) mindestens einen blauen direktziehenden Farbstoff der allgemeinen Formel (III) und ii) mindestens einen gelben direktziehenden Farbstoff der Formel (I) und/oder mindestens einen roten direktziehenden Farbstoff der Formel (II) sowie iii) gegebenenfalls weitere direktziehende und/oder oxidative Farbstoffvorstufen enthält, 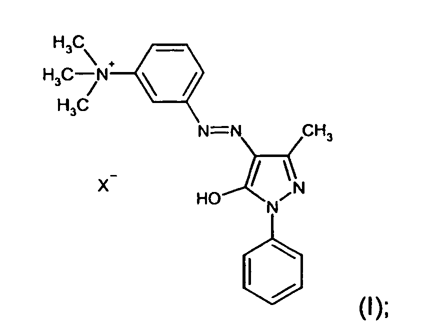 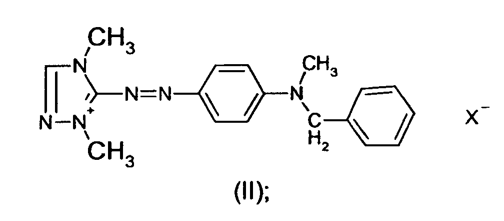 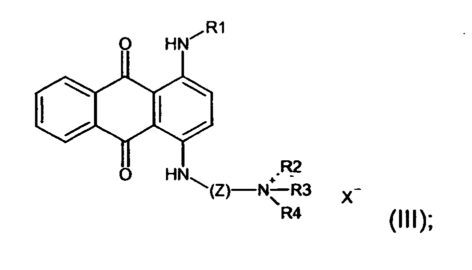 worin **R1** ein Wasserstoffatom, eine (C1-C4)-Alkylgruppe, eine Phenylgruppe oder eine (C2-C4)-Hydroxyalkylgruppe darstellt;
**R2, R3** und **R4** gleich oder verschieden sein können und eine (C1-C4)-Alkylgruppe darstellen; **Z** ein (C2-C6)-Alkylen-Diradikal darstellt und
**X**^{**-**} für ein Anion aus der Gruppe Chlorid, Bromid, Alkylsulfat, Arylsulfat, Sulfat oder Acetat steht.

10. Mittel nach Anspruch 9, **dadurch gekennzeichnet, dass** der Farbstoff der Formel (I) das 3-((4,5-Dihydro-3-methyl-5-oxo-1-phenyl-1H-pyrazol-4-yl)azo)-N,N,N-trimethyl-benzenaminium-chlorid beziehungsweise das 3-[(3-Methyl-5-hydroxy-1-phenyl-1H-pyrazol-4-yl)azo]-trimethylammonio-benzol-chlorid ist.

11. Mittel nach Anspruch 9, **dadurch gekennzeichnet, dass** der Farbstoff der Formel (II) das 3(oder5)-[[4-[Benzylmethylamino]phenyl]-azo]-1,2-(oder 1,4)-dimethyl-1,2,4-triazolium-bromid ist.

12. Mittel nach Anspruch 9, **dadurch gekennzeichnet, dass** der Farbstoff der Formel (III) ausgewählt ist aus N,N,N-Trimethyl-3-{[4-(methylamino)-9,10-dioxo-9,10-dihydro-1-anthracenyl]amino}-1-propanaminium-methylsulfat, N,N-Dimethyl-3-{[4-(methylamino)-9,10-dioxo-9,10-dihydro-1-anthracenyl]amino}-N-propyl-1-propanaminium-chlorid und N,N-Dimethyl-3-{[4-(methylamino)-9,10-dioxo-9,10-dihydro-1-anthracenyl]amino}-N-propyl-1-propanaminium-bromid.

13. Mittel nach einem der Ansprüche 1 bis 12, **dadurch gekennzeichnet, dass** es ein Haarfärbemittel ist.

14. Verfahren zum Färben von Keratinfasern, bei dem eine für die Färbung ausreichende Menge eines Färbemittels auf das Haar aufgetragen wird und das Haar nach einer Einwirkungszeit von 10 bis 45 Minuten bei 15 bis 50 °C mit Wasser ausgespült und gegebenenfalls getrocknet wird, **dadurch gekennzeichnet, dass** ein Färbemittel nach einem der Ansprüche 1 bis 13 verwendet wird.

15. Verfahren zum Färben von Keratinfasern, **dadurch gekennzeichnet, dass** in einer ersten Stufe ein Oxidationsmittel-freies Färbemittel (Farbträgermasse), enthaltend i) mindestens einen blauen direktziehenden Farbstoff der allgemeinen Formel (III) und ii) mindestens einen gelben direktziehenden Farbstoff der Formel (I) und/oder mindestens einen roten direktziehenden Farbstoff der Formel (II) sowie iii) gegebenenfalls weitere Farbstoffe, 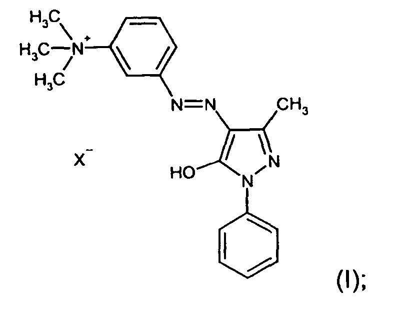 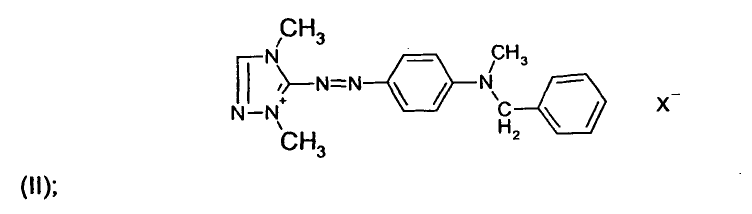 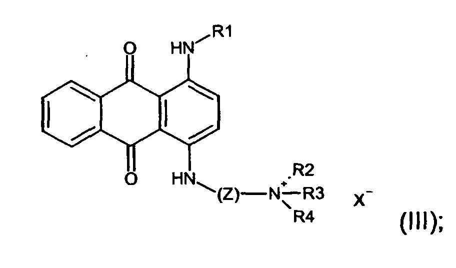 worin **R1** ein Wasserstoffatom, eine (C1-C4)-Alkylgruppe, eine Phenylgruppe oder eine (C2-C4)-Hydroxyalkylgruppe darstellt;
**R2, R3** und **R4** gleich oder verschieden sein können und eine (C1-C4)-Alkylgruppe darstellen; **Z** ein (C2-C6)-Alkylen-Diradikal darstellt und
**X-** für ein Anion aus der Gruppe Chlorid, Bromid, Alkylsulfat, Arylsulfat, Sulfat oder Acetat steht,
auf die durch Oxidationsmittel bereits vorgeschädigten Haarpartien aufgetragen wird, und in einer zweiten Stufe die vorgenannte Farbträgermasse zunächst mit einem Oxidationsmittel vermischt wird und sodann auf das restliche Haar aufgetragen wird.

16. Verfahren nach Anspruch 14 oder 15, **dadurch gekennzeichnet, dass** die Farbträgermasse mit dem Oxidationsmittel in einem Gewichtsverhältnis von 5:1 bis 1:3 vermischt wird.

## Claims

1. Ready-to-use composition for the lightening and dyeing of keratin fibres, **characterized in that** it comprises (a) an oxidizing agent and (b) at least one blue direct-dyeing colorant of the general formula (III) and (c) at least one yellow direct-dyeing colorant of the formula (I) and/or at least one red direct-dyeing colorant of the formula (II), and (d) has a basic pH 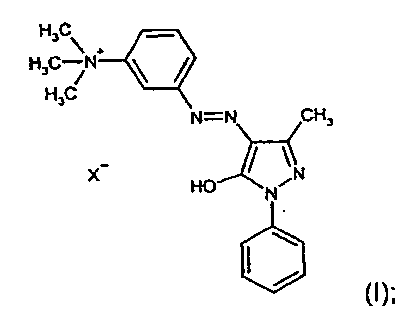 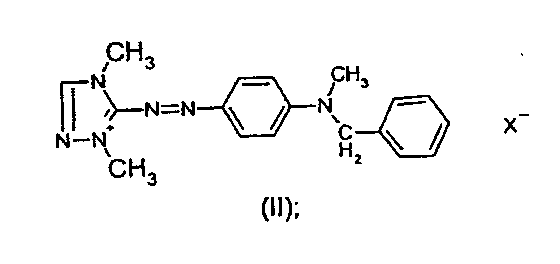 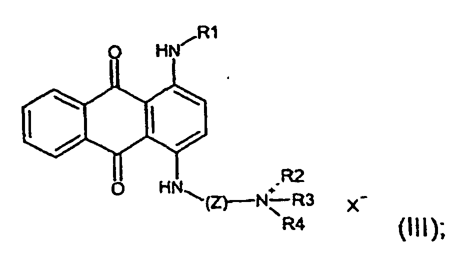 in which **R1** is a hydrogen atom, a (C1-C4)-alkyl group, a phenyl group or a (C2-C4)-hydroxyalkyl group, **R2, R3** and **R4** may be identical or different and are a (C1-C4)-alkyl group; **Z** is a (C2-C6)-alkylene diradical and **X**^{**-**} is an anion from the group consisting of chloride, bromide, alkylsulphate, arylsulphate, sulphate or acetate.

2. Composition according to Claim 1, **characterized in that** the colorant of the formula (III) is chosen from compounds of the formula (III) in which **R1** is a (C1-C4)-alkyl group, the radicals **R2** and **R3** are a methyl group and **R4** is a (C1-C4)-alkyl group; **Z** corresponds to a C2- or C3-akylene diradical and **X**^{**-**} is an anion from the group consisting of chloride, bromide or alkylsulphate.

3. Composition according to Claim 1, **characterized in that** the colorant of the formula (I) is 3-((4,5-dihydro-3-methyl-5-oxo-1-phenyl-1H-pyrazol-4-yl)azo)-N,N,N-trimethylbenzenaminium chloride or 3-[(3-methyl-5-hydroxy-1-phenyl-1H-pyrazol-4-yl)azo]trimethyl-ammoniobenzene chloride.

4. Composition according to Claim 1, **characterized in that** the colorant of the formula (II) is 3(or 5)-[[4-[benzylmethylamino]phenyl]azo]-1,2(or 1,4)-dimethyl-1,2,4-triazolium bromide.

5. Composition according to one of Claims 1 to 4, **characterized in that** the colorant of the formula (III) is chosen from N,N,N-trimethyl-3-{[4-(methylamino)-9,10-dioxo-9,10-dihydro-1-anthracenyl]amino}-1-propanaminium methylsulphate, N,N-dimethyl-3-{[4-(methylamino)-9,10-dioxo-9,10-dihydro-1-anthracenyl]amino}-N-propyl-1-propanaminium chloride and N,N-dimethyl-3-{[4-(methylamino)-9,10-dioxo-9,10-dihydro-1-anthracenyl]-amino}-N-propyl-1-propanaminium bromide.

6. Composition according to one of Claims 1 to 5, **characterized in that** the total content of the colorants of the formula (I) to (III) is 0.001 to 8 per cent by weight.

7. Composition according to one of Claims 1 to 6, **characterized in that**, in addition to the colorants of the formulae (I) to (III), it comprises further direct-dyeing and/or oxidative colorant precursors.

8. Composition according to one of Claims 1 to 7, **characterized in that** the ready-to-use dye has a pH of from 8 to 11.

9. 2-Component composition for dyeing keratin fibres, consisting of a colour carrier mass (A) and a component (B) comprising an oxidizing agent, **characterized in that** the colour carrier mass (A) comprises i) at least one blue direct-dyeing colorant of the general formula (III) and ii) at least one yellow direct-dyeing colorant of the formula (I) and/or at least one red direct-dyeing colorant of the formula (II) and iii) optionally further direct-dyeing and/or oxidative colorant precursors, 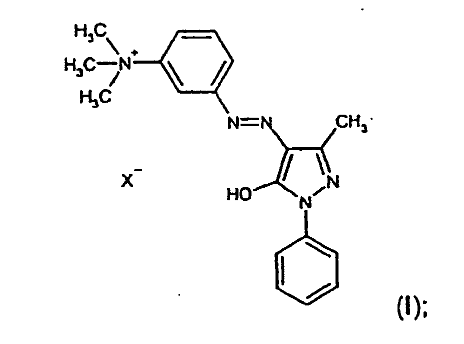 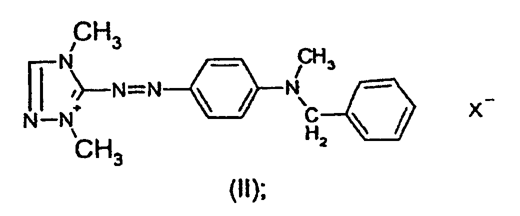 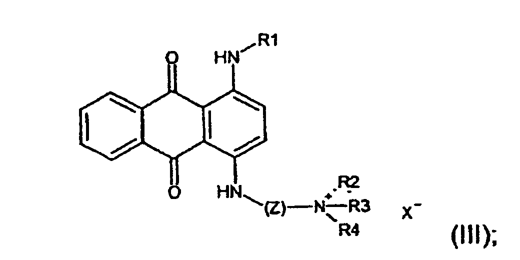 in which **R1** is a hydrogen atom, a (C1-C4)-alkyl group, a phenyl group or a (C2-C4)-hydroxyalkyl group; **R2, R3** and **R4** may be identical or different and are a (C1-C4)-alkyl group; **Z** is a (C2-C6)-alkylene diradical and **X-** is an anion from the group consisting of chloride, bromide, alkylsulphate, arylsulphate, sulphate or acetate.

10. Composition according to Claim 9, **characterized in that** the colorant of the formula (I) is 3-((4,5-dihydro-3-methyl-5-oxo-1-phenyl-1H-pyrazol-4-yl)azo)-N,N,N-trimethylbenzenaminium chloride or 3-[(3-methyl-5-hydroxy-1-phenyl-1H-pyrazol-4-yl)azo]trimethylammoniobenzene chloride.

11. Composition according to Claim 9, **characterized in that** the colorant of the formula (II) is 3(or 5)-[[4-[benzylmethylamino]phenyl]azo]-1,2(or 1,4)-dimethyl-1,2,4-triazolium bromide.

12. Composition according to Claim 9, **characterized in that** the colorant of the formula (III) is chosen from N,N,N-trimethyl-3-{[4-(methylamino)-9,10-dioxo-9,10-dihydro-1-anthracenyl]amino}-1-propanaminium methylsulphate, N,N-dimethyl-3-{[4-(methylamino)-9,10-dioxo-9,10-dihydro-1-anthracenyl]amino}-N-propyl-1-propanaminium chloride and N,N-dimethyl-3-{[4-(methylamino)-9,10-dioxo-9,10-dihydro-1-anthracenyl]amino}-N-propyl-1-propanaminium bromide.

13. Composition according to one of Claims 1 to 12, **characterized in that** it is a hair dye.

14. Method of dyeing keratin fibres in which an amount of a dye sufficient for the dyeing is applied to the hair and, after a contact time of from 10 to 45 minutes at 15 to 50°C, the hair is rinsed with water and optionally dried, **characterized in that** a dye according to one of Claims 1 to 13 is used.

15. Method of dyeing keratin fibres, **characterized in that**, in a first stage, an oxidizing agent-free dye (colour carrier mass), comprising i) at least one blue direct-dyeing colorant of the general formula (III) and ii) at least one yellow direct-dyeing colorant of the formula (I) and/or at least one red direct-dyeing colorant of the formula (II) and iii) optionally further colorants, 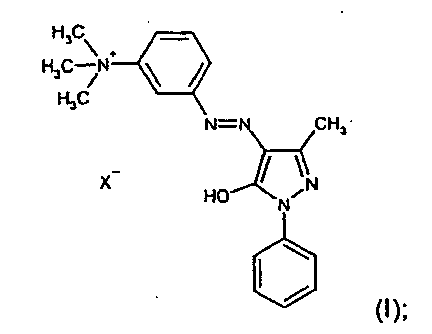 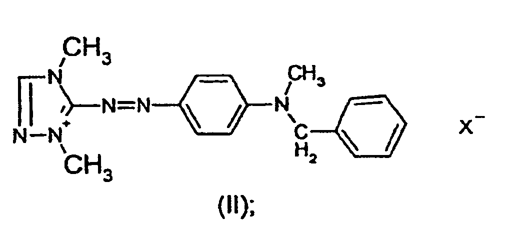 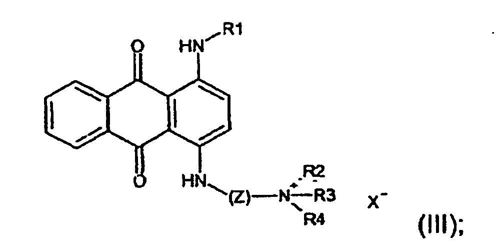 in which **R1** is a hydrogen atom, a (C1-C4)-alkyl group, a phenyl group or a (C2-C4)-hydroxyalkyl group; **R2, R3** and **R4** may be identical or different and are a (C1-C4)-alkyl group; **Z** is a (C2-C6)-alkylene diradical and **X**^{**-**} is an anion from the group consisting of chloride, bromide, alkylsulphate, arylsulphate, sulphate or acetate,
is applied to the sections of hair already predamaged by oxidizing agents, and in a second stage the abovementioned colour carrier mass is firstly mixed with an oxidizing agent and is then applied to the rest of the hair.

16. Method according to Claim 14 or 15, **characterized in that** the colour carrier mass is mixed with the oxidizing agent in a weight ratio of from 5:1 to 1:3.

## Revendications

1. Composition pour l'éclaircissement et la teinture de fibres de kératine, **caractérisée en ce qu'**elle contient (a) un oxydant ainsi que (b) au moins un colorant direct bleu de formule générale (III) et (c) au moins un colorant direct jaune de formule (I) et/ou au moins un colorant direct rouge de formule (II), et (d) présente un pH basique, 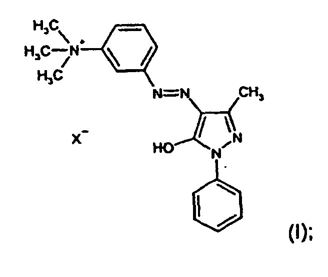 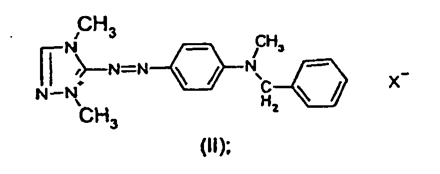 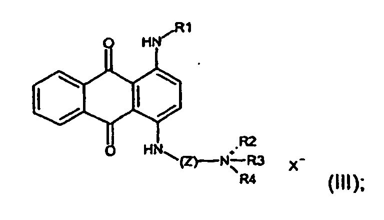 formules dans lesquelles **R1** représente un atome d'hydrogène, un groupe alkyle en C₁-C₄, un groupe phényle ou un groupe hydroxyalkyle en C₂-C₄ ; **R2, R3** et **R4** peuvent être identiques ou différents et représentent un groupe alkyle en C₁-C₄ ; **Z** représente un diradical alkylène en C₂-C₆ et **X**^{**-**} représente un anion choisi dans le groupe constitué par les anions chlorure, bromure, alkylsulfate, arylsulfate, sulfate et acétate.

2. Composition selon la revendication 1, **caractérisée en ce que** le colorant de formule (III) est choisi parmi des composés de formule (III) dans lesquels **R1** est un groupe alkyle en C₁-C₄, les radicaux **R2** et **R3** représentent un groupe méthyle et **R4** représente un groupe alkyle en C₁-C₄ ; **Z** correspond à un diradical alkylène en C₂ ou C₃ et
**X**^{**-**} représente un anion choisi dans le groupe constitué par les anions chlorure, bromure et alkylsulfate.

3. Composition selon la revendication 1, **caractérisée en ce que** le colorant de formule (I) est le chlorure de 3-((4,5-dihydro-3-méthyl-5-oxo-1-phényl-1H-pyrazol-4-yl)azo)-N,N,N-triméthyl-benzène-aminium ou le chlorure de 3-[(3-méthyl-5-hydroxy-1-phényl-1H-pyrazol-4-yl)azo]-triméthylammonio-benzène.

4. Composition selon la revendication 1, **caractérisée en ce que** le colorant de formule (II) est le bromure de 3(ou 5)-[[4-[benzylméthylamino]-phényl]azo]-1,2-(ou 1,4)-diméthyl-1,2,4-triazolium.

5. Composition selon l'une quelconque des revendications 1 à 4, **caractérisée en ce que** le colorant de formule (III) est choisi parmi le méthylsulfate de N,N,N-triméthyl-3-{[4-(méthylamino)-9,10-dioxo-9,10-dihydro-1-anthracényl]-amino}-1-propanaminium, le chlorure de N,N-diméthyl-3-{[4-(méthylamino)-9,10-dioxo-9,10-dihydro-1-anthracényl]amino}-N-propyl-1-propanaminium et le bromure de N,N-diméthyl-3-{[4-(méthylamino)-9,10-dioxo-9,10-dihydro-1-anthracényl]amino}-N-propyl-1-propanaminium.

6. Composition selon l'une quelconque des revendications 1 à 5, **caractérisée en ce que** la teneur totale en les colorants de formules (I) à (III) va de 0,001 à 8 % en poids.

7. Composition selon l'une quelconque des revendications 1 à 6, **caractérisée en ce qu'**en plus des colorants de formules (I) à (III) elle contient d'autres précurseurs de colorants directs et/ou d'oxydation.

8. Composition selon l'une quelconque des revendications 1 à 7, **caractérisée en ce que** la composition de teinture prête à l'emploi présente un pH de 8 à 11.

9. Composition à deux composants pour la teinture de fibres de kératine, constituée d'une matière colorante (A) et d'un composant (B) contenant un oxydant, **caractérisée en ce que** la matière colorante (A) contient (i) au moins un colorant direct bleu de formule générale (III) et (ii) au moins un colorant direct jaune de formule (I) et/ou au moins un colorant direct rouge de formule (II) ainsi que (iii) éventuellement d'autres précurseurs de colorants directs et/ou d'oxydation, 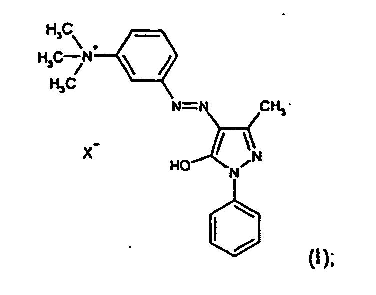 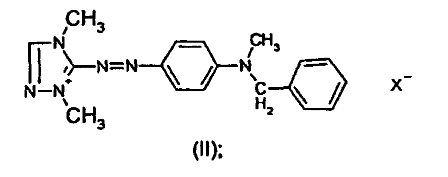 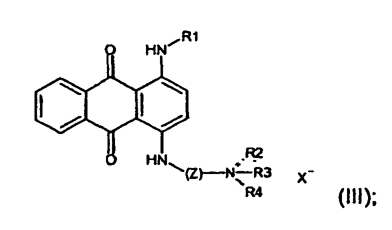 formules dans lesquelles **R1** représente un atome d'hydrogène, un groupe alkyle en C₁-C₄, un groupe phényle ou un groupe hydroxyalkyle en C₂-C₄ ; **R2, R3** et **R4** peuvent être identiques ou différents et représentent un groupe alkyle en C₁-C₄ ; **Z** représente un diradical alkylène en C₂-C₆ et **X**^{**-**} représente un anion choisi dans le groupe constitué par les anions chlorure, bromure, alkylsulfate, arylsulfate, sulfate et acétate.

10. Composition selon la revendication 9, **caractérisée en ce que** le colorant de formule (I) est le chlorure de 3-((4,5-dihydro-3-méthyl-5-oxo-1-phényl-1H-pyrazol-4-yl)azo)-N,N,N-triméthylbenzène-aminium ou le chlorure de 3-[(3-méthyl-5-hydroxy-1-phényl-1H-pyrazol-4-yl)azo]-triméthylammonio-benzène.

11. Composition selon la revendication 9, **caractérisée en ce que** le colorant de formule (II) est le bromure de 3(ou 5)-[[4-[benzylméthylamino]-phényl]azo]-1,2-(ou 1,4)-diméthyl-1,2,4-triazolium.

12. Composition selon la revendication 9, **caractérisée en ce que** le colorant de formule (III) est choisi parmi le méthylsulfate de N,N,N-triméthyl-3-{[4-(méthylamino}-9,10-dioxo-9,10-dihydro-1-anthracényl]amino)-1-propanaminium, le chlorure de N,N-diméthyl-3-{[4-(méthylamino)-9,10-dioxo-9,10-dihydro-1-anthracényl]amino}-N-propyl-1-propanaminium et le bromure de N,N-diméthyl-3-{[4-(méthylamino)-9,10-dioxo-9,10-dihydro-1-anthracényl]amino}-N-propyl-1-propanaminium.

13. Composition selon l'une quelconque des revendications 1 à 12, **caractérisée en ce qu'**il s'agit d'une composition de teinture pour cheveux.

14. Procédé pour la teinture de fibres de kératine, dans lequel on applique sur les cheveux une quantité, suffisante pour la teinture, d'une composition de teinture et, après un temps d'action de 10 à 45 minutes à 15-50°C, on rince les cheveux à l'eau et éventuellement on les sèche, **caractérisé en ce qu'**on utilise une composition de teinture selon l'une quelconque des revendications 1 à 13.

15. Procédé pour la teinture de fibres de kératine, **caractérisé en ce que** dans une première étape on applique sur les parties des cheveux déjà préalablement abîmées par des oxydants une composition de teinture sans oxydant (matière colorante), contenant (i) au moins un colorant direct bleu de formule générale (III) et (ii) au moins un colorant direct jaune de formule (I) et/ou au moins un colorant direct rouge de formule (II) ainsi que (iii) éventuellement d'autres colorants, 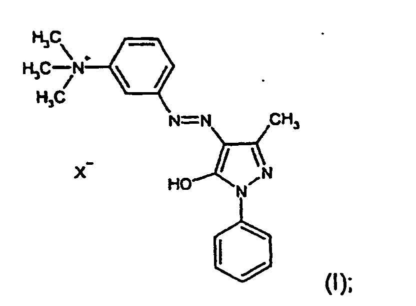 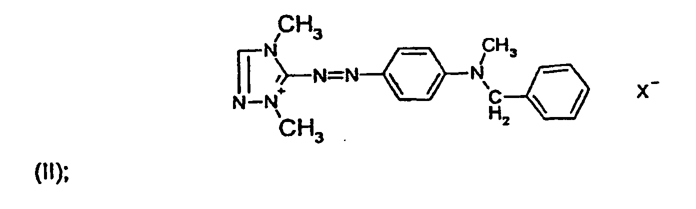 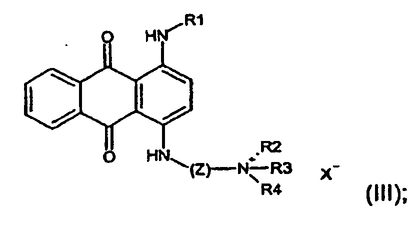 formules dans lesquelles **R1** représente un atome d'hydrogène, un groupe alkyle en C₁-C₄, un groupe phényle ou un groupe hydroxyalkyle en C₂-C₄ ; **R2, R3** et **R4** peuvent être identiques ou différents et représentent un groupe alkyle en C₁-C₄ ; **Z** représente un diradical alkylène en C₂-C₆ et **X**^{**-**} représente un anion choisi dans le groupe constitué par les anions chlorure, bromure, alkylsulfate, arylsulfate, sulfate et acétate, et dans une deuxième étape d'abord on mélange la matière colorante précitée avec un oxydant et ensuite on applique ce mélange sur les cheveux restants.

16. Procédé selon la revendication 14 ou 15, **caractérisé en ce qu'**on mélange la matière colorante avec l'oxydant en un rapport pondéral de 5:1 à 1:3.
